(19) Europäisches Patentamt
European Patent Office
Office européen des brevets

(11) Publication number: **0 146 050 B1**

(12) **EUROPEAN PATENT SPECIFICATION**

(45) Date of publication of patent specification: **04.07.90**

(51) Int. Cl.[5]: **C 12 N 11/02**, C 12 N 15/00, C 12 P 21/00, A 61 K 39/395, A 61 K 37/54

(21) Application number: **84114547.7**

(22) Date of filing: **30.11.84**

# (54) Site selective plasminogen activator and method of making and using same.

(30) Priority: **16.12.83 US 562464**
**19.11.84 US 673198**

(43) Date of publication of application:
**26.06.85 Bulletin 85/26**

(45) Publication of the grant of the patent:
**04.07.90 Bulletin 90/27**

(84) Designated Contracting States:
**BE DE FR GB IT SE**

(56) References cited:
**SCIENCE, vol. 222, 09.12.1983, pp.1129-1132, (US); K.Y. HUY et al.: "Monoclonal antibodies to a synthetic fibrin-like peptide bind to human fibrin but not fibrinogen"**

**NATURE, vol. 290, no. 5802, 12.03.1981, pp.145,146, CHESHAM, BUCKS (GB); H.E. BLYTHMAN et al.:"Immunotoxins: hybrid molecules of monoclonal antibodies and a toxin subunit specifically kill tumour cells"**

(73) Proprietor: **Callewaert, Denis M.**
**1600 Hosner Road**
**Oxford Michigan 48051 (US)**

(72) Inventor: **Callewaert, Denis M.**
**1600 Hosner Road**
**Oxford Michigan 48051 (US)**

(74) Representative: **Dipl.-Ing. H. Hauck Dipl.-Phys. W. Schmitz Dipl.-Ing. E. Graalfs Dipl.-Ing. W. Wehnert Dr.-Ing. W. Döring**
**Mozartstrasse 23**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 146 050 B1

Courier Press, Leamington Spa, England.

## Description

### Technical Field

The present invention relates to the urokinase and streptokinase antibody conjugates capable of activating plasminogen in animal blood (including human blood) and effective on administration to deliver plasminogen activator activity selectively to a specific site where fibrinolysis is desired. The present invention further relates to the method of making such conjugates and to their therapeutic use.

### Background of the Invention

Urokinase is a known plasminogen activator. It has received clinical use, particularly for the treatment of pulmonary embolism, myocardial infarcts, deep vein thrombosis, and peripheral arterial occlusion. However, urokinase in the available forms is relatively unselective as to its site of activity. In order to administer sufficient urokinase to lyse a localized fibrin clot, such as a clot localized in a coronary artery, heavy dosage far exceeding the amount ultimately effective at the desired reaction site is required. Further, urokinase is characterized by a very short half life in the environment of the circulatory system, of the order of roughly 10 minutes. This increases the amount (and cost) required for effective dosage still further. While proposals and some potential applications of antibody techniques to target drugs generally to antigens on specific cells have been made, application of the general technique to target urokinase to fibrin clots has not been previously attempted. Several reasons are believed to account for this. The antibody targeting techniques proposed or developed to date have been largely, if not entirely, limited to relatively simple and directly operating active materials. Thus, for example, Vitetta et al in "Immunotoxins: A New Approach to Cancer Therapy" Science 219, 644, describes experiments using ricin as a toxic moiety, and lists various other toxic moieties. Such moieties, although sometimes enzymatic in nature and therefore complex and rather readily destroyed, operate directly on the target material (e.g. a cancer cell) or otherwise involve a mechanism of operation very different from that of urokinase. Urokinase action, however, is quite different since it can operate only indirectly to destroy fibrin by activating the circulating proenzyme plasminogen. Any urokinase-antibody coupled material must not only activate the circulating plasminogen but in addition it must be free from interference with the fibrinolytic action of plasmin. The short half-life of urokinase in the blood stream presents an obstacle not predictably overcome by antibody techniques. In any event, I am not aware of any significant belief that antibody techniques can be applied to urokinase to achieve useful clinical applications.

Another known plasminogen activator is streptokinase. While streptokinase reactions in the blood stream, including the need to overcome antibodies to streptokinase, are not identical to those of urokinase, the principles of the present invention are nevertheless applicable to streptokinase.

Unlike the prior workers of whom I have knowledge, I concluded that monoclonal antibodies specific for fibrin or myosin can be coupled with urokinase so as to produce a site selective urokinase action in activating plasminogen without interfering with the action of plasmin, and thereby achieve urokinase (or streptokinase) therapy with small and therefore clinically useful doses in man and animals.

### Summary

The present invention involves the production of antibodies to human or other animal fibrin or myosin by administration to mice or other suitable animals which are later sacrificed and the antibody producing cells obtained from their spleens. The resulting antibody producing cells are thereupon fused with myeloma cells of the same species, and the desired resultant cells are subcloned to obtain a continuous or immortal cell line producing the desired monoclonal antibody. A conjugate of such monoclonal antibody is made with urokinase (or streptokinase) to provide both the plasminogen activating activity of urokinase (or streptokinase) and site-specificity together, surprisingly with greatly prolonged lifetime in human serum.

### Detailed Description

In accordance with the present invention, antibody is prepared by immunizing an animal of a second species (e.g. mouse) with fibrin fragments or cardiac myosin of the human or animal to be treated. Myeloma cells of the second species are fused with spleen cells from the immunized second species to provide antibody producing immortal hybrid cells. The hybrid cells are cloned, selected, and subcloned to provide hybrid cells that produce antibody specific to the target protein and non-specific to fibrinogen. The antibody is isolated from the subclone culture medium, or otherwise, and coupled with urokinase (or streptokinase) to produce the site-specific plasminogen activator complex of the present invention. Specific illustrative techniques for accomplishing these steps are set forth in the following examples:

### Example I

Preparation of Immunogens to Elicit Anti-human Fibrin Antibodies via Amino-terminal Disulfide Knot Fragment, (T) N—DSK, From Human Fibrin

Commercially available high purity human fibrinogen is converted to fibrin by the action of thrombin. The fibrin is dissolved in 70% formic acid to a protein concentration of one percent. Cyanogen bromide (1.3 g/100 ml) is added with stirring at room temperature, and the mixture is allowed to react for 24 hours. The solution is evaporated using a rotary evaporator, and the residue is taken up in 10% acetic acid for gel

filtration in Sephadex G-100 equilibrated with 10% acetic acid. The (T) N—DSK fractions are concentrated and further purified by ion exchange chromatography on DEAE-Sephadex using a salt gradient. These procedures follow Blomback et al (J. Biol. Chem *247,* 1496 (1972): J. Biol. Chem. *248,* 5806 (1973)) and Kudryk et al (Molecular Immunology *20,* 1191 (1983)).

Example II

Preparation of Immunogens to Elicit Anti-human Fibrin Antibodies via Fibrin Peptide-maleimidobenzoylated Keyhole Limpet Hemocyanin Conjugate (Fibrin Peptide-MB—KLH)

A heptapeptide with an amino acid sequence identical to the amino terminal end of the beta-chain of human fibrin is chemically synthesized by the standard Merrifield solid phase method (R. B. Merrifield, J. Am. Chem. Soc. *95,* 2149 (1962); J. M. Stewart, J. D. Young, *Solid Phase Peptide Synthesis,* c. 1969 Freeman, San Francisco, pp. 27—62). A cysteinyl residue is attached to the carboxyl terminal lysyl residue of the peptide in order to allow the peptide to be covalently attached to the maleimidobenzoylated keyhole limpet hemocyanin (MB—KLH). Synthetic peptides with a specific sequence such as this fibrin-like peptide are commercially available from companies such as Research Plus Labs., Inc. of Bayonne, New Jersey. For the coupling procedure, specifically, five milligrams of keyhold limpet hemocyanin (KLH) in 0.25 ml of 0.05 M phosphate buffer are reacted with m-maleimidobenzoyl-N-hydroxysuccinimide ester (MBS) (5 milligrams MBS/milliter tetrahydrofuran) at a KLH/MBS ratio of 1/40 for 30 minutes at room temperature. The resulting MB—KLH intermediate is isolated by gel filtration chromatography on Sephadex G-25. Five milligrams of the synthetic peptide in 0.5 ml of 0.1 M phosphate buffer is mixed with MB—KLH and incubated for 3 hours at room temperature. The product of the reaction is the immunogen, fibrin peptide-MB—KLH. This procedure is described by Kwan H. Hui et al (Science *222,* 1129—32 (1983).

Example III

Preparation of Immunogen to Elicit Anti-human Cardiac Myosin Antibodies

Human cardiac myosin is purified from the human heart by application of the procedure described for purification of canine cardiac myosin (J. Wikman-Coffelt et al, Biochem. Biophys. Res. Commun. *51,* 1097—1104 (1973)). The procedure involves homogenizing human heart tissue in phosphate buffer, fractionating the same by centrifugation, adding ammonium sulfate, and crystallizing myosin from dilute ionic solution. The resultant crystallized product is used as the immunogen.

Example IV

Preparation of Splenocytes

In one procedure to produce splenocytes, BALB/c mice are each injected intraperitoneally with a first, 100-microgram dose of immunogen prepared, for example, in accordance with one of the above examples. The immunogen is suspended in a 1:1 mixture of phosphate buffered saline (PBS) and Freund's complete adjuvant; a second dose is administered one month later. Three days following an intravenous booster injection of the immunogen, the mice are sacrificed and their spleens removed. A single-cell suspension of splenocytes is prepared by pressing the spleen tissue through a stainless steel mesh.

The above are illustrations of conventional, known, procedures for obtaining splenocytes having antibody activity to human fibrin or myosin, which per se and by themselves form no part of the present invention. As an illustrative description of such procedure see, for example, Kwan H. Hui et al [Science, 222, 1129—32 (1983)] and B. Kudryk et al [Molecular Immunology *21,* 89 (1984)]. Further, the description is limited to the mouse as the antibody producing animal, although other animals such as rats or humans may be chosen.

Example V

Production of Monoclonal Antibodies to Human Fibrin or Myosin

Spenocytes produced, for example, as above, are fused with the mouse myeloma cell line P3-NSI/4-AG-4-1. Illustratively, the following modification of the fusion procedure of Erlich et al [J. Immunol., *128,* 2709—2713 (1982)] may be used. Specifically, 200 million splenocytes are fused with 20 million myeloma cells in 0.5 ml of fusion medium comprising 35% polyethylene glycol 1000 and 5% dimethylsulfoxide in RPMI 1640 medium. After cell fusion, the cells are cultured for several weeks in a medium containing hypoxanthine, aminopterin and thymidine (HAT) at 37°C in an atmosphere containing 5% carbon dioxide. Hybrid colonies are then screened for antibody production by removing a 50-microliter aliquot of the culture supernates and adding this to microtiter wells containing either polymerized fibrin or adsorbed fibrinogen, in the case of fibrin-immunized cells, and cardiac myosin, in the case of myosin-immunized cells.

After incubation for 30—90 minutes at 37°C, the plates are washed and a preparation of enzyme-coupled goat antimouse immunoglobulin (Ig-peroxidase) is added, the plates are incubated at 37°C. and washed again, and a chromogenic substrate mixture for the peroxidase (such as 0.05 M sodium phosphate — 0.024 M citrate buffer, pH 5.0, containing 0.04% o-phenylenediamine and 0.012% hydrogen peroxide) is added to the wells. The qualitative and quantitative presence of antibody to either fibrin (or myosin) and fibrinogen is thereupon monitored by the intensity of the color produced upon further incubation with the substrate mixture. Colonies that are determined to produce antibody to fibrin (or myosin) but not to

fibrinogen are cloned, and the cloned colonies retested for antibody production to fibrin (or myosin). Clones that are positive for antibody to fibrin fragments but negative to antibody to purified fibrinogen or clones recognizing the myosin H chain but not L chain are subcloned and grown in tissue culture. Samples of all suitable clones are also frozen for later use.

The cloning procedures, per se, above described are taught by Kohler and Milstein [Nature, *256*, 495—497 (1975)] and in more recent publications. Monoclonal antibody technology has now advanced to the point where these reagents are produced and can be used for a variety of purposes [see P. C. Kung and G. Goldstein, U.S. Patent 4,381,295 (1983)]. The specific known cloning techniques above described do not, per se and by themselves, form any part of the present invention. Of course, if animals other than mice are used to generate antibodies, the same animal species must generally be the source of the myeloma from which the hybridoma is derived.

Example VI

Conjugation with Urokinase Using SPDP

Urokinase and rabbit anti-human fibrinogen have been successfully coupled using as the coupling reagent N-succinimidyl 3-(2-pyridyldithio) propionate (SPDP). This is a heterobifunctional reagent developed by Carlsson and associates [Protein Thiolation and Reversible Protein-Protein Conjugation. N-Succinimidyl 3-(2-pyridyldithio) propionate, a New Heterobifunctional Reagent. Biochem. J. *173*, 723 (1978). It can react with both amino groups and sulfhydryl groups of proteins. This type of reagent decreases the amount of intramolecular cross-linking that can occur — particularly when one of the proteins to be conjugated lacks either free sulfhydryl or free amino groups. Urokinase has free amino groups but lacks free sulfhydryl groups, and so the SPDP reacts with the urokinase to form derivatives with one or more 3-(2-pyridyldithio)propionyl groups covalently attached per molecule of urokinase through amino residues of the urokinase. The successful reaction of urokinase and SPDP yields 3-(2-pyridyldithio)-propionyl-urokinase (PDTP—UK). Before mixing with the rabbit anti-human fibrinogen, the PDTP—UK was extensively dialyzed to remove excess SPDP. The rabbit anti-human fibrinogen (RAHF) had been mildly reduced with dithiothreitol to expose sulfydryl groups in the antibody molecule. It is believed that the reaction of the rabbit anti-human fibrinogen and PDTP—UK should produce, and did produce:

$$UK{-}NH{-}\overset{\overset{\displaystyle O}{\|}}{C}{-}CH_2{-}CH_2{-}S{-}S{-}RAHF$$

The SPDP coupling procedure used in the above successful conjugation of urokinase (UK) and rabbit anti-human fibrinogen (RAHF) was a modification of procedures developed by Hi-Her Jau [Methods in Enzymology *92*, J. J. Lagone and H. Van Vanakis, Academic Press N.Y. (1983), p. 257] and Pharmacia Fine Chemicals [SPDP Brief Instructions Leaflet, Pharmacia Fine Chemicals, Piscataway, New Jersey]. Approximately 100,000 CTA units of urokinase in 0.5 ml of 0.1 M sodium phosphate buffer, pH 7.2, containing 0.15 sodium chloride were reacted with 0.015 ml of 10 mM SPDP (in ethanol) for 30 minutes at room temperature. The mixture was dialized overnight vs. 0.1 M sodium phosphate buffer, pH 7.5, containing 0.15 M sodium chloride (PBS) at 4 degrees C. The antibody to fibrinogen, 9 mg/ml in PBS, was partially reduced by incubation in 0.1 M dithiothreitol for one hour at room temperature. After gel filtration on Sephadex G-25 to remove excess dithiothreitol, 1.7 mg of the reduced antibody was mixed with the dialyzed PDTP—UK intermediate, and this mixture was incubated for 24 hours at room temperature. The resultant reaction mixture after incubation contained the desired urokinase-antibody material. Tests of the urokinase activity of the reaction mixture above have shown that about 22% of the original urokinase activity is recoverable. The conjugate was separated from unreacted material by gel filtration chromatography using Sephadex G-200.

The urokinase-antifibrinogen conjugate was examined to determine the extent to which functional rabbit anti-human fibrinogen antibody was present and to further characterize the urokinase activity. Microtiter plate wells were coated with human fibrinogen by incubating the plates with a solution of fibrinogen in phosphate buffered saline. The plates were thoroughly rinsed to remove any unbound fibrinogen, and then either a sample of the conjugate or mixtures of uncoupled rabbit anti-human fibrinogen and urokinase were added to the fibrinogen coated wells. After 90 minutes incubation at 37°C, the plates were again extensively washed with phosphate buffered saline containing 0.05% Tween 20. The wells were examined for: (1) urokinase amidase activity using the general technique described in D. Paar and D. Marukn, J. Clin. Chem. Clin. Biochem. *18*, 557 (1980), (2) urokinase plasminogen activator activity using the general technique described in R. C. Wohl, C. Summaria, and K. C. Robbins, Jl. Biol. Chem. *255*, 2005 (1980), and (3) bound rabbit anti-human fibrinogen. The urokinase amidase activity was measured by using the relatively urokinase selective substrate S-2444 (pyroglutamyl-glycyl-arginyl-p-nitroanilide). The hydrolysis of S-2444 by urokinase was monitored by observing the increase in absorption at 405 nm that results from the release of p-nitroaniline from the S-2444. The results of these assays are given in Table I, below. Uncoupled urokinase or up to 600 times more rabbit anti-human fibrinogen antibody than was calculated to be present in the complex were tested in control wells that were washed in the same manner as the wells containing the urokinase-anti-human fibrinogen complex. The washed control wells did not

contain detectable amounts of the rabbit immunoglobulin and were essentially negative for urokinase activity as shown in table I.

Table I — Urokinse Amidase Activity of Urokinase Rabbit Anti-Human Fibrinogen

| Material Tested | Fibrinogen Wells*+ | Uncoated Wells+ |
|---|---|---|
| Urokinase-Rabbit-Anti-Human Fibrinogen Ab conjugate | 0.299 +/- 0.011 | 0.071 +/- 0.009 |
| Rabbit Anti-Human Fibrinogen Ab | 0.053 +/- 0.004 | 0.055 +/- 0.003 |
| Rabbit Anti-Human Fibrinogen Ab and Urokinase | 0.087 +/- 0.003 | 0.084 +/- 0.004 |
| Urokinase | 0.082 +/- 0.002 | 0.066 +/- 0.004 |

*Wells of microtiter plates are coated with 2 ug of fibrinogen before proceeding with the addition of the materials tested.

+Absorbance at 405nm.

The urokinase plasminogen activator activity of the urokinase-rabbit anti-human fibrinogen complex was also determined by adding plasminogen to the wells, and then after a 15 minute incubation determining the plasmin activity by adding the plasmin substrate S-2251 (H-D-val-leu-lys-p-nitroanilide) to the wells. As with the S-2444, the hydrolysis of S-2251 was monitored by observing the increase in absorption at 405 nm. The results demonstrate that the complex retains plasminogen activator activity.

The stability of urokinase in plasma, that is, the inhibition of unmodified urokinase by plasma has been reported. See E. K. Waller et al, Biochem. J. *215,* 123 (1983); G. Murano et al, Blood *55,* 430 (1980) [inhibition of urokinase by plasma] and M. Verstraete in *Fibrinolysis,* ed. D. L. Kline and K. N. N. Reddy, CRC Press, Cleveland, OH. (1980), p. 187 [clearance of urokinase from blood]. Of particular importance, tests have indicated that the stability of urokinase-antibody material in human plasma is much greater than that of unmodified urokinase. In these tests, human blood was collected using heparin as the anticoagulant (2000 units of heparin per 50 ml of whole blood), and the formed elements were removed by centrifugation. Urokinase or the urokinase-rabbit anti-human fibrinogen was incubated with the plasma at 37°C. Aliquots, containing comparable units of urokinase activity, were removed after incubation for varying times, and the urokinase activity remaining was determined using the small amidase substrate, L-pyroglu-gly-arg-p-nitroanilide. The results showed that urokinase is inactivated considerably more rapidly and to a greater extent than is the urokinase-rabbit anti-human fibrinogen complex.

In particular tests have shown that after 3 hours of incubation, approximately 30 percent of the urokinase activity remains for uncoupled urokinase incubated in vitro with human plasma. Under the same conditions approximately 80 percent of the urokinase activity of conjugate made as above described remained. While in vitro and in vivo conditions differ (in part because there may be actual removal or clearance of the protein of interest in the blood in vivo), a positive correlation exists between stability of urokinase in vitro in plasma, and the half life in vivo. See E. K. Waller et al, Biochem. J. *215,* 123 (1983); G. Murano et al, Blood *55,* 430 (1980) and M. Verstraete in *Fibrinolysis,* ed. D. L. Kline and K. N. N. Reddy, CRC Press, Cleveland, OH. (1980), p. 187.

It is believed that coupling agents having a function that reacts with the amino groups of the urokinase are most effective for urokinase-antibody conjugate formation. Thiol groups can be introduced into the urokinase as was done with the heterofunctional agent SPDP, but the initial reaction involves amino groups of the urokinase. While experimental conditions may be found that allow homobifunctional groups such as glutaraldehyde to form active conjugates, it is believed that intramolecular reactions greatly reduce or eliminate the enzymatic activity of the resulting conjugates. Thus in accordance with the preferred embodiment of the present invention, the coupling agent is a heterobifunctional agent such as SPDP or

succinimidyl-4-(p-maleimidophenyl) butyrate, SMPB, which reacts with amino groups of the urokinase and then with sulfhydryl groups of the antibody. The function of the coupling agent that reacts with the sulfhydryl group of the antibody can thus be a disulfide as in SPDP, a maleimide as in SMPB or some other group with similar activity. Successful coupling of other enzymes to antibodies demonstrates the great flexibility of the antibody molecules in the coupling reactions. Antibody specificity has been retained when coupling is through either amino or sulfhydryl groups of the antibody protein. Changes in the spacer group between the functional groups of bifunctional coupling agents appear to be less critical in nature and many variations are possible that will still allow the formation of an active urokinase-antibody conjugate.

For a review of bifunctional reagents which have been used to achieve chemical cross-links of protein, see Han, et al, Int. J. Biochem. *16,* pp. 129—145 (1984). In the application of any such reagent (preferably selected as above described to provide a function that reacts with amino groups of the urokinase), the reaction conditions such as concentrations, temperatures, time, pH, and the like need to be selected to achieve effective coupling. For example, we have found undesirable precipitation and inactivity in initial experiments using glutaraldehyde as the coupling agent. No urokinase activity was detected in the high molecular weight fractions (where coupled material would be expected) when urokinase and antibody coupling was attempted using the coupling agent N,N'-o-phenylene dimaleimide.

Example VII

Coupling of Streptokinase to Rabbit Anti-human Fibrinogen

To demonstrate application of this invention to streptokinase, the following experiment was performed. Streptokinase from B-Hemolytic streptococcus was obtained from Sigma Chemical Company in the form of a lyophilized powder containing 50 percent human serum albumin as a stabilizing agent. Albumin was removed by Affi-gel Blue chromatography (Castellino, F. J., Sodetz, J. M., Brockway, W. J., Siefring, G. E., Jr., Streptokinase, in *Methods in Enzymology 45,* Ed. Laszlo Lorand, Academic Press, N.Y. (1976), p. 244). The two stage assay procedure used to measure streptokinase action was substantially as described by Jackson, Esmon and Tang (Streptokinase and Staphylokinase, in *Methods in Enzymology 80,* Ed. Laszlo Lorand, Academic Press, N.Y. (1981), p. 387). In this method, the plasmin specific substrate D-val-leu-lys-p-nitroanilide (Kabi S2251) is used. The coupling procedure using SPDP was essentially the same as above described with respect to urokinase and rabbit anti-human fibrinogen. The lyophilized purified streptokinase in the concentration of 1.6 mg in 0.7 ml of phosphate buffered saline was mixed with 0.01 ml of a 10 mM ethanol solution of SPDP. The mixture was incubated at room temperature for 30 minutes and then dialyzed vs. one liter of phosphate buffered saline at 4°C overnight.

Rabbit antibody to human fibrinogen was pretreated with dithiothreitol, dialyzed, combined with the dialyzed PDTP-streptokinase, and the mixture was incubated overnight at room temperature. The reaction mixture was then chromatographed using Sephadex G-200. Approximately 5 percent of the initial streptokinase activity was recovered from the Sephadex G-200 column, with about 3 percent of the activity being present as a streptokinase anti-human fibrinogen conjugate.

Using the same microtiter plate technique as described above for determining activity of the urokinase conjugate, the streptokinase anti-human-fibrinogen conjugate was found to bind to human fibrinogen and to retain plasminogenic activating capability.

**Claims**

1. A process of producing monoclonal antibody-enzyme complex selected from the enzymes urokinase and streptokinase specific for target protein selected from fibrin and cardiac myosin of an animal species and characterized by relatively prolonged urokinase half-life in plasma, comprising the steps of immunizing an animal of a second species with the target protein, fusing myeloma cells of the second species with spleen cells from the thus immunized second species to provide antibody producing immortal hybrid cells, cloning the hybrid cells, selecting and subcloning in a culture medium those of the hybrid cells that produce antibody that is specific to said target protein and non-specific to fibrinogen, isolating the thus produced antibody, and coupling the monoclonal antibody with the selected enzyme.

2. The process according to claim 1 where the enzyme is urokinase.

3. The process of claim 2 where the animal of the second species is a mouse.

4. The process of claim 1 where the coupling agent has groups reactive with the amino groups of the urokinase.

5. The process of claim 2 where the coupling agent is N-succinimidyl 3-(2-pyridyldithio) propionate (SPDP).

6. The process of claim 2 where the coupling agent is succinimidyl-4-(p-maleimidophenyl) butyrate (SMPB).

7. As a therapeutic product suitable for lysing fibrin in site-selective action in an animal species identified by a target protein selected from fibrin and cardiac myosin, the reaction product of immunizing an animal of a second species with the target protein, fusing myeloma cells of the species with spleen cells from the thus immunized second species to provide antibody producing immortal hybrid cells, cloning the hybrid cells, selecting and subcloning in a culture medium those of the hybrid cells that produce antibody that is specific to said target protein and non-specific to fibrinogen, isolating the thus produced antibody,

and coupling the monoclonal antibody with an enzyme selected from the group consisting of urokinase and streptokinase.

8. The therapeutic product of claim 7 where the enzyme is urokinase and the coupling agent has groups reactive with the amino groups of the urokinase.

9. The therapeutic product of claim 7 where the coupling agent is SPDP.

10. The therapeutic product of claim 7 where the coupling agent is SMPB.

**Patentansprüche**

1. Verfahren zur Herstellung eines monoklonalen Antikörper-Enzym-Komplexes, der aus den Enzymen Urokinase und Streptokinase ausgewählt ist, welche für ein Target-Protein spezifisch sind, das aus Fibrin und Herz-Myosin einer Tierspezies ausgewählt ist, und der durch eine verlängerte Urokinase-Halbwertszeit im Plasma gekennzeichnet ist, mit den folgenden Schritten: Immunisieren eines Tieres einer zweiten Spezies mit dem Target-Protein, Verschmelzen von Myelom-Zellen der zweiten Spezies mit Milz-Zellen der auf diese Weise immunisierten zweiten Spezies, um unsterbliche, Antikörper erzeugende Hybrid-Zellen vorzusehen, Klonen der Hybrid-Zellen, Auswählen und Subklonen von denjenigen der Hybrid-Zellen, die Antikörper erzeugen, welche in bezug auf das Target-Protein spezifisch und in bezug auf Fibrinogen nicht spezifisch sind, in einem Kulturmedium, Isolieren der auf diese Weise erzeugten Antikörper und Koppeln des monoklonalen Antikörpers mit dem ausgewählten Enzym.

2. Verfahren nach Anspruch 1, bei dem das Enzym Urokinase ist.

3. Verfahren nach Anspruch 2, bei dem das Tier der zweiten Spezies eine Maus ist.

4. Verfahren nach Anspruch 1, bei dem das Kopplungsmittel Gruppen besitzt, die mit den Aminogruppen der Urokinase reaktiv sind.

5. Verfahren nach Anspruch 2, bei dem das Kopplungsmittel N-Succinimidyl-3-(2-Pyridylthio) Propionat (SPDP) ist.

6. Verfahren nach Anspruch 2, bei dem das Kopplungsmittel Succinimidyl-4-(p-Maleimido-Phenyl) Butyrat (SMPB) ist.

7. Therapeutisches Produkt, das zum Lösen von Fibrin in stellenselektiver Wirkung in einer Tierspezies, identifiziert durch ein aus Fibrin und Herz-Myosin ausgewähltes Target-Protein, geeignet ist und das das aus den nachfolgenden Schritten gewonnene Reaktionsprodukt darstellt: Immunisieren eines Tieres einer zweiten Spezies mit dem Target-Protein, Verschmelzen von Myelom-Zellen der Spezies mit Milzzellen der auf diese Weise immunisierten zweiten Spezies, um unsterbliche, Antikörper erzeugende Hybrid-Zellen vorzusehen, Klonen der Hybrid-Zellen, Auswählen und Subklonen von denjenigen der Hybrid-Zellen, die Antikörper erzeugen, die in bezug auf das Target-Protein spezifisch und in bezug auf Fibrinogen nicht spezifisch sind, in einem Kulturmedium, Isolieren der auf diese Weise erzeugten Antikörper und Koppeln der monoklonalen Antikörper mit einem Enzym, das aus der aus Urokinase und Streptokinase bestehenden Gruppe ausgewählt ist.

8. Therapeutisches Produkt nach Anspruch 7, bei dem das Enzym Urokinase ist und das Kopplungsmittel Gruppen besitzt, die mit den Aminogruppen der Urokinase reaktiv sind.

9. Therapeutisches Produkt nach Anspruch 7, bei dem das Kopplungsmittel SPDP ist.

10. Therapeutisches Produkt nach Anspruch 7, bei dem das Kopplungsmittel SMPB ist.

**Revendications**

1. Procédé de production du complexe d'un anticorps monoclonal et d'une enzyme sélectionnée parmi l'urokinase et la streptokinase, spécifique à l'égard d'une protéine-cible choisie parmi la fibrine et la myosine cardiaque d'une espèce animale et caractérisé en ce que l'urokinase possède un temps de demivie dans le plasma relativement prolongé, comportant comme étapes l'immunisation d'un animal d'une seconde espèce avec la protéine-cible, la fusion de cellules de myélome de la seconde espèce avec des cellules de la rate de la seconde espèce immunisée de la sorte afin de produire des cellules hybrides immortelles de production d'anticorps, le clonage des cellules hybrides, la sélection et le sous-clonage dans un milieu de culture des cellules hybrides produisant un anticorps qui soit spécifique de ladite protéine-cible et non spécifique à l'égard du fibrinogène, l'isolement de l'anticorps produit de la sorte et la couplage de l'anticorps monoclonal avec l'enzyme sélectionnée.

2. Procédé selon la revendication 1, caractérisé en ce que l'enzyme est l'urokinase.

3. Procédé selon la revendication 2, caractérisé en ce que l'animal de seconde espèce est une souris.

4. Procédé selon la revendication 1, caractérisé en ce que l'agent de couplage possède des substituants réagissant avec les groupes amino de l'urokinase.

5. Procédé selon la revendication 2, caractérisé en ce que l'agent de couplage est le 3-(2-pyrydyldithio) propionate de N-succinimidyle.

6. Procédé selon la revendication 2, caractérisé en ce que l'agent de couplage est le 4-(p-maléimidophényl) butyrate de succinimidyle (SMPB).

7. Comme substance thérapeutique appropriée pour lyser de la fibrine en une action sélective et propre à un site déterminé, dans une espèce animale identifiée par une protéine-cible choisie parmi la fibrine et la myosine cardiaque, le produit de la réaction comportant l'immunisation d'un animal de la seconde espèce

avec la protéine-cible, la fusion des cellules de myélome de l'espèce avec des cellules de la rate de la seconde espèce immunisée de la sorte afin de produire des cellules hybrides immortelles de production d'anticorps, le clonage des cellules hybrides, la sélection et le sous-clonage dans un milieu de culture des cellules hybrides produisant un anticorps qui soit spécifique de ladite protéine-cible et non spécifique à l'égard du fibrinogène, l'isolement de l'anticorps produit de la sorte et le couplage de l'anticorps monoclonal avec une enzyme sélectionnée dans le groupe formé par l'urokinase et la streptokinase.

8. Substance thérapeutique selon la revendication 7, caractérisée en ce que l'enzyme est l'urokinase et l'agent de couplage possède des substituants réagissant avec les groupes amino de l'urokinase.

9. Substance thérapeutique selon la revendication 7, caractérisée en ce que l'agent de couplage est le SPDP.

10. Substance thérapeutique selon la revendication 7, caractérisée en ce que l'agent de couplage est le SMPB.